# Europäisches Patentamt

# European Patent Office

# Office européen des brevets

(19)

(11) Veröffentlichungsnummer: **0 056 496**
**B1**

(12) **EUROPÄISCHE PATENTSCHRIFT**

(45) Veröffentlichungstag der Patentschrift:
27.06.84

(51) Int. Cl.³: **C 07 D 213/10**

(21) Anmeldenummer: 81110832.3

(22) Anmeldetag: 29.12.81

(54) Verfahren zur Herstellung von 3-Picolin.

(30) Priorität: 09.01.81 CH 104/81
12.03.81 CH 1685/81

(73) Patentinhaber: **LONZA AG, Gampel/Wallis (CH)**

(43) Veröffentlichungstag der Anmeldung:
28.07.82 Patentblatt 82/30

(72) Erfinder: **Dinkel, Rolf, Dr., Lärchenstrasse 8,
Münchenstein Kt. Baselland (CH)**
Erfinder: **Roedel, Hilmar, Dr., Bienenweg 6, Therwil Kt.
Baselland (CH)**
Erfinder: **Grayson, James Ian, Dr., Kleegärtenstrasse 1,
Visp, Kt. Wallis (CH)**

(45) Bekanntmachung des Hinweises auf die Patenterteilung:
27.06.84 Patentblatt 84/26

(74) Vertreter: **von Füner, Alexander, Dr. et al, Patentanwälte
v. Füner, Ebbinghaus, Finck Mariahilfplatz 2 & 3,
D-8000 München 90 (DE)**

(84) Benannte Vertragsstaaten:
AT BE CH DE FR GB IT LI LU NL SE

(56) Entgegenhaltungen:
EP - A - 0 040 698
DE - A - 1 695 296
DE - A - 2 337 087

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

## Beschreibung

Die vorliegende Erfindung betrifft ein Verfahren zur Herstellung von 3-Picolin.

Pyridinbasen stellen wichtige Zwischenprodukte in der chemischen Industrie dar, so z. B. bei der Herstellung von Nicotinsäure oder Nicotinsäureamid. Es sind verschiedene Verfahren zur Herstellung von Pyridinbasen bekannt.

2-Methyl-5-Ethylpyridin wird heute großtechnisch im Flüssigphasenverfahren aus Acetaldehyd oder Paraldehyd und Ammoniak in Gegenwart verschiedenster Katalysatoren, wie z. B. Ammoniumsalze, hergestellt. Als Nebenprodukt fallen kleine Mengen an 2- und 4-Picolin an.

2- und 4-Picolin werden heute in Gasphasenreaktionen bei Temperaturen von ca. 400°C aus Acetaldehyd und Ammoniak unter Verwendung von Festbett- oder Fließbettkatalysatoren auf Basis Aluminiumsilikat hergestellt (siehe Chemical Economy & Engineering Review, June 1975, No. 6, Seite 34 und 35).

Für die Erzeugung von Pyridin sowie 3-Picolin, welches immer größere Bedeutung bekommt, werden heute Gasphasenreaktionen angewendet, wobei durch Zugabe von Formaldehyd zum Acetaldehyd die Bildung von 2- und 4-Picolin zu Gunsten von 3-Picolin unterdrückt wird. Auch diese Umsetzungen finden im Festbett oder Fließbett mit Aluminiumsilikat als Katalysator bei Temperaturen von etwa 400°C statt. Nach diesen Verfahren werden Ausbeuten an 3-Picolin in der Größenordnung von höchstens 40 bis 44% erzielt. Daneben fallen große Mengen an Pyridin an.

Es ist auch bekannt, daß anstelle von gesättigten Aldehyden von ungesättigten Aldehyden, wie z. B. Acrolein oder Crotonaldehyd, ausgegangen werden kann. Diese Reaktionen finden in der Gasphase bei hohen Temperaturen statt; die Ausbeuten liegen im wesentlichen gleich hoch wie bei der Verwendung von gesättigten Aldehyden als Ausgangsmaterial (siehe Chem. Techn., 12. Jg., Heft 12, Dezember 1970, Seite 745 bis 748, Hans Joachim Übel, Karl-Klaus Moll und Manfred Mühlstädt, Untersuchungen zur Gewinnung von 3-Methylpyridin).

Ziel der vorliegenden Erfindung ist es, 3-Picolin in hohen Ausbeuten herzustellen, wobei die Bildung von Pyridin möglichst unterdrückt werden soll.

Erfindungsgemäß wird dies dadurch erreicht, daß man Acetaldehydacetale, gegebenenfalls im Gemisch mit Acetaldehyd bzw. dessen Polymeren und/oder Crotonaldehyd mit Formaldehydacetalen und/oder Hexamethylentetramin, gegebenenfalls im Gemisch mit Formaldehyd bzw. dessen Polymeren in wässeriger Phase bei einem Molverhältnis von Acetaldehydacetalen und gegebenenfalls Acetaldehyd zu Formaldehydacetalen und gegebenenfalls Formaldehyd von 1 : 0,5 bis 1 : 1,2 bzw. Crotonaldehyd zu Formaldehydacetalen, gegebenenfalls unter Zusatz von Formaldehyd von 1 : 1 bis 1 : 2,4 sowie Acetaldehydacetale, gegebenenfalls mit Acetaldehyd zu Hexamethylentetramin von 1 : 0,083 bis 1 : 0,2 bei Temperaturen von 180 bis 280°C im geschlossenen Gefäß in Gegenwart von Ammoniak und/oder Ammoniumionen und in Gegenwart von anorganischen oder organischen Säuren, die bei 20°C eine Dissoziationskonstante von $10^6$ bis $10^{-12}$ aufweisen, umsetzt.

Unter Acetaldehyd im Sinne der Erfindung sind auch dessen Polymere, wie z. B. Paraldehyd, zu verstehen; unter Formaldehyd auch dessen Polymere, wie z. B. Trioxan.

Acetaldehydacetale/Acetaldehyd und/oder Crotonaldehyd wird im folgenden als Edukt 1 und Formaldehydacetale/Formaldehyd und/oder Hexamethylentetramin als Edukt 2 bezeichnet.

Vorzugsweise gelangen die einzelnen möglichen Komponenten der Edukte 1 und 2 jeweils nur einzeln zum Einsatz. So beim Edukt 1 ein Acetaldehydacetal und gegebenenfalls Acetaldehyd, oder Crotonaldehyd und beim Edukt 2 ein Formaldehydacetal und gegebenenfalls Formaldehyd, oder Hexamethylentetramin.

Um die für die Reaktion wichtigen Anionen von anorganischen und/oder organischen Säuren, die bei 20°C eine Säure-Dissoziationskonstante von $10^6$ bis $10^{-12}$ aufweisen, in die Reaktionslösung einzubringen, werden die entsprechenden wasserlöslichen Alkali- und/oder Ammoniumsalze dieser Säuren der Reaktionslösung zugesetzt.

Salze der Säuren im Sinne vorliegender Erfindung sind beispielsweise Natrium-, Kalium- oder Ammoniumsalze

| | |
|---|---|
| der Pentaborsäure | wie Ammoniumpentaborat, |
| der Kohlensäure | wie Ammoniumcarbonat, |
| der Phosphorsäure | wie Kaliumdihydrogenphospat, Ammoniumdihydrogenphosphat, Dikaliumhydrogenphosphat oder Diammoniumhydrogenphosphat, |
| der Schwefelsäure | wie Natriumhydrogensulfat oder Ammoniumsulfat, |
| der Fluorsäure | wie Natriumfluorid, Ammoniumfluorid oder Ammoniumhydrogendifluorid, |
| der Salzsäure | wie Ammoniumchlorid, |
| der Bromwasserstoffsäure | wie Ammoniumbromid, |
| der Heptamolybdänsäure | wie Ammoniumheptamolybdat, |
| der Ameisensäure | wie Ammoniumformiat, |
| der Essigsäure | wie Natriumacetat oder Ammoniumacetat, |
| der Propionsäure | wie Ammoniumpropionat, |

| der Buttersäure | wie Ammoniumbutyrat, |
| der Bernsteinsäure | wie Dinatriumsuccinat oder Diammoniumsuccinat, |
| der Adipinsäure | wie Diammoniumadipinat, |
| der Benzoesäure | wie Natriumbenzoat oder Ammoniumbenzoat, |
| der Phthalsäure | wie Diammoniumphthalat, |
| der Terephthalsäure | wie Diammoniumterephthalat, |
| der Nikotinsäure | wie Ammoniumnikotinat sowie |
| der Isonikotinsäure | wie Ammoniumisonikotinat. |

Für die Bildung von 3-Picolin aus Acetaldehyd und/oder Acetaldehydacetalen und/oder Crotonaldehyd mit Formaldehyd und/oder Formaldehydacetalen und/oder Hexamethylentetramin ist die Anwesenheit von Ammoniak und/oder Ammoniumionen notwendig. Wird Ammoniak angewendet, das entweder gasförmig oder als wässerige Lösung eingesetzt werden kann, genügt es, wenn Alkalisalze der genannten Säuren eingesetzt werden. Es können aber auch Gemische von Alkalisalzen und Ammoniumsalzen eingesetzt werden. Wird auf Ammoniak verzichtet, sind Ammoniumsalze oder Gemische von Ammoniumsalzen und Alkalisalzen zu verwenden. Werden miteinander nicht mischbare flüssige Ausgangsmaterialien, wie z. B. Paraldehyd zusammen mit wässerigem Formaldehyd, verwendet, so ist es vorteilhaft, zur Homogenisierung kleine Mengen Homogenisierungsmittel, wie Alkohole, cyclische Äther, vorzugsweise jedoch vorgebildetes 3-Picolin, einzusetzen, oder die nicht mischbaren flüssigen Ausgangsmaterialien mit je einer eigenen Pumpe in den Reaktor einzuspeisen.

Nach dem Verfahren der Erfindung wird überraschenderweise 3-Picolin in Ausbeuten bis ca. 65% erhalten und die Bildung von Pyridin fast vollständig unterdrückt (unter 1%). Als Nebenprodukt fallen 3-Ethylpyridin sowie kleine Mengen an 2,5-Dimethylpyridin, 3,5-Dimethylpyridin und 2-Methyl-5-ethylpyridin an.

Das Verfahren der Erfindung wird mit einem Molverhältnis Acetaldehydacetale und gegebenenfalls Acetaldehyd zu Formaldehydacetale und gegebenenfalls Formaldehyd von 1 : 0,5 bis 1 : 1,2, vorzugsweise 1 : 0,8 bis 1 : 1, durchgeführt.

Wird Crotonaldehyd anstelle von Acetaldehydacetalen und Acetaldehyd verwendet, so verschiebt sich das Molverhältnis Crotonaldehyd zu Formaldehyd und Formaldehydacetalen entsprechend auf 1 : 1 bis 1 : 2,4.

Wird Hexamethylentetramin anstelle von Formaldehyd und gegebenenfalls Formaldehydacetalen verwendet, so verschiebt sich das Molverhältnis Acetaldehydacetale/Acetaldehyd zu Hexamethylentetramin entsprechend auf 1 : 0,083 bis 1 : 0,2.

Die Reaktionstemperaturen liegen bei 180 bis 280°C, zweckmäßig bei 205 bis 240°C, vorzugsweise bei 225 bis 235°C. Die Reaktion wird in flüssiger Phase (wässeriger Phase) unter einem Druck, der sich bei der Reaktion im geschlossenen Gefäß bei vorgegebener Temperatur einstellt, durchgeführt. Es ist vorteilhaft, während der Reaktion den Reaktionsansatz zu rühren.

Die Menge an Ammoniak und/oder Ammoniumionen liegen bei 0,5 bis 3 Mol Ammoniak und/oder Ammoniumionen pro Mol Edukt, zweckmäßig bei 0,5 bis 2,0 Mol pro Mol Edukt.

Die Menge an Anionen von anorganischen und/oder organischen Säuren liegt zweckmäßig bei 0,1 bis 3 Mol, vorzugsweise bei 0,2 bis 1,0 Mol pro Mol Edukt.

Die Ammoniumsalze werden vorzugsweise in wässeriger Lösung in einer Konzentration von 0,3 bis 10 Mol-% angewendet.

Der Start-pH-Wert der wässerigen Reaktionslösung liegt zweckmäßig zwischen 5,0 und 12,5.

Die Zugabe des Aldehyds erfolgt zweckmäßig nach Maßgabe seines Verbrauches. So ist es beispielsweise günstig, beim Arbeiten in einem 2-Liter-Behälter und bei Einsatz von 350 ml Aldehyd diesen kontinuierlich während 30 bis 90 Minuten zuzusetzen. Bei anderen Bedingungen sind die entsprechenden Zugabezeiten zu wählen.

Am Ende der gewünschten Reaktionsperiode wird die Temperatur auf etwa Raumtemperatur gesenkt und das 3-Picolin auf bekannte Weise aus der Reaktionsmischung gewonnen. Eine Methode besteht darin, daß man den pH-Wert der Wasserphase zuerst in den basischen Bereich bringt und dann das organische Material aus der wäßrigen Reaktionsmischung mit einem organischen Lösungsmittel, z. B. Benzol, Toluol, Xylol, Methylenchlorid, Chloroform, Äther und dergleichen, extrahiert. Das organische Lösungsmittel wird dann abgedampft und man erhält 3-Picolin durch fraktionierte Destillation. Im Rahmen der Erfindung können auch beliebige andere Methoden zur Abtrennung und Gewinnung des Produktes angewendet werden.

Ein Vorteil des neuen erfindungsgemäßen Verfahrens ist auch der, daß die nach der Extraktion der Reaktionsmischung mit einem organischen Lösungsmittel erhaltene wäßrige Phase nach Wiederanreicherung mit Ammoniak und/oder Ammoniumionen in den Reaktor zurückgeführt werden kann. Die wäßrige Salzphase ist zusammengesetzt aus der ursprünglich in der Salzlösung vorhandenen Wassermenge, der nicht umgesetzten Menge an Ammoniak und/oder Ammoniumsalz, dem gegebenenfalls vorhandenen Metallsalz, der freigesetzten Säure des an der Umsetzung teilnehmenden Ammoniumsalzes sowie einem Mol Wasser für jedes Mol bei der Umsetzung verbrauchten Eduktes. Die wäßrige Salzphase wird daher mit Hilfe eines beliebigen bekannten Verfahrens konzentriert, z. B. durch Verdampfen, um das infolge der Kondensationsreaktion gebildete Wasser zu entfernen.

**0 056 496**

Eine Wiederanreicherung von Ammoniak und/oder Ammoniumsalz wird dann dadurch erreicht, daß man in die wäßrige Lösung bei Umgebungstemperatur gasförmigen Ammoniak einbringt unter Rückbildung des Ammoniumsalzes aus der gegebenenfalls vorhandenen Säure.

Obgleich die Erfindung als diskontinuierliches Verfahren beschrieben worden ist, kann das Verfahren auch im Rahmen der vorliegenden Erfindung kontinuierlich betrieben werden. Bei einer Ausführungsform eines kontinuierlichen Verfahrens werden die Reaktionsteilnehmer kontinuierlich in einen geeigneten Druckreaktor eingeführt, aus dem die Reaktionsmischung kontinuierlich abgezogen wird. Die Reaktionsprodukte werden daraus abgetrennt, die wäßrige Salzphase aufkonzentriert und unveränderte Reaktionsteilnehmer werden dann wieder ergänzt und in das Reaktionsgefäß zurückgeführt.

Das kontinuierliche Verfahren kann in jedem Reaktor durchgeführt werden, der eine innige Vermischung der Reaktionsteilnehmer unter heftigem Rühren gestattet, z. B. in einem kontinuierlich gerührten Tankreaktor.

Beispiel 1

1140 ml einer 3,40molaren wäßrigen Lösung von Diammoniumhydrogenphosphat (pH 8,35) wurden in einem 2-Liter-Autoklaven auf 230°C erhitzt und bei 1500 UpM gerührt. In diese Lösung wurde innert 60 Min. kontinuierlich ein Gemisch aus 117,7 g Paraldehyd, 49,8 g Hexamethylentetramin, 130 g Wasser und 120 g Ethanol eingepumpt (kalkulatorisches Molverhältnis Acetaldehyd zu Formaldehyd = 1 : 0,80). Dabei variierte der Reaktionsdruck zwischen 35 und 44 bar. Nach beendeter Zugabe des Eduktgemisches wurde die Reaktionsmasse während 10 Min. bei 230°C weitergerührt und dann auf Raumtemperatur abgekühlt. Schließlich erfolgte eine Extraktion mit 3 · 100 ml Methylenchlorid sowie eine gaschromatographische Analyse der vereinigten Methylenchloridextrakte, wobei sich folgende Produkte mit den je nach Aldehyd-Bedarf auf eingesetzten Paraldehyd (A) oder auf eingesetztes Hexamethylentetramin (F) bezogenen Ausbeuten ergaben: Pyridin 0,8% (A); 3-Picolin 65,6% (F), 3-Ethylpyridin 19,0% (A); 2,5-Lutidin 3,4% (A); 3,5 Lutidin 0,8% (F); 2-Methyl-5-ethylpyridin 2,8% (A). Die wäßrige Phase hatte einen pH von 9,4 nach der Extraktion.

Beispiel 2

1140 ml einer 3,40molaren wäßrigen Lösung von Diammoniumhydrogenphosphat (pH 8,35) wurden in einem 2-Liter-Autoklaven auf 220°C erhitzt und bei 1500 UpM gerührt. In diese Lösung wurde innert 59 Min. ein Gemisch aus 120,0 g Paraldehyd, 49,8 g Hexamethylentetramin, 120 g Wasser und 120 g Ethanol eingepumpt (kalkulatorisches Molverhältnis Acetaldehyd zu Formaldehyd = 1 : 0,79). Dabei variierte der Reaktionsdruck zwischen 26 und 40 bar. Nach beendeter Zugabe des Eduktgemisches wurde die Reaktionsmasse während 10 Min.bei 220°C weitergerührt und dann auf Raumtemperatur abgekühlt. Schließlich erfolgte eine Extraktion mit 3 · 100 ml Methylenchlorid sowie eine gaschromatographische Analyse der vereinigten Methylenchloridextrakte, wobei sich folgende Produkte mit den je nach Aldehyd-Bedarf auf eingesetzten Paraldehyd (A) oder auf eingesetztes Hexamethylentetramin (F) bezogenen Ausbeuten ergaben: Pyridin 0,3% (A); 3-Picolin 51,4% (F); 3-Ethylpyridin 6,0% (A); 2,5-Lutidin 1,4% (A); 3,5-Lutidin 2,7% (F); 2-Methyl-5-ethylpyridin 6,8% (A). Die wäßrige Phase hatte einen pH von 9,3 nach der Extraktion.

Beispiel 3

1140 ml einer 3,40molaren wäßrigen Lösung von Diammoniumhydrogenphosphat (pH 8,35) wurden in einem 2-Liter-Autoklaven auf 230°C erhitzt und bei 1500 UpM gerührt. In diese Lösung wurde innert 64 Min. kontinuierlich ein Gemisch aus 117,7 g Paraldehyd und 162,2 g Formaldehyddimethylacetal eingepumpt (kalkulatorisches Molverhältnis Acetaldehyd zu Formaldehyd = 1 : 0,74). Dabei variierte der Reaktionsdruck zwischen 33 und 40 bar. Nach beendeter Zugabe des Eduktgemisches wurde die Reaktionsmasse während 10 Min. bei 230°C weitergerührt und dann auf Raumtemperatur abgekühlt. Schließlich erfolgte eine Extraktion mit 3 · 100 ml Methylenchlorid sowie eine gaschromatographische Analyse der vereinigten Methylenchloridextrakte, wobei sich folgende Produkte mit den je nach Aldehyd-Bedarf auf eingesetzten Paraldehyd (A) oder auf eingesetztes Formaldehyddimethylacetal (F) bezogenen Ausbeuten ergaben: Pyridin 1,0% (A); 3-Picolin 53,6% (F); 3-Ethylpyridin 23,9% (A); 2,5-Lutidin 4,3% (A) ; 3,5-Lutidin 0,6% (F); 2-Methyl-5-ethylpyridin 2,5% (A).

Beispiel 4

1140 ml einer 3,40molaren wäßrigen Ammoniumacetat-Lösung (pH 7,55) wurden in einem 2-Luter-Autoklaven auf 230°C erhitzt und bei 1500 UpM gerührt. In diese Lösung wurde innert 51 Min. kontinu-

4

ierlich ein Gemisch aus 96,0 g Paraldehyd, 39,8 g Hexamethylentetramin, 104 g Wasser und 100 g Ethanol eingepumpt (kalkulatorisches Molverhältnis Acetaldehyd zu Formaldehyd = 1 : 0,80). Dabei variierte der Reaktionsdruck zwischen 25 und 32 bar. Nach beendeter Zugabe des Eduktgemisches wurde die Reaktionsmasse während 10 Min. bei 230°C weitergerührt und dann auf Raumtemperatur abgekühlt. Schließlich erfolgte eine Extraktion mit 3 · 100 ml Methylenchlorid sowie eine gaschromatographische Analyse der vereinigten Methylenchloridextrakte, wobei sich folgende Produkte mit den je nach Aldehyd-Bedarf auf eingesetzten Paraldehyd (A) oder auf eingesetztes Hexamethylentetramin (F) bezogenen Ausbeuten ergaben: Pyridin 1,0% (A); 3-Picolin 56,3% (F); 3-Ethylpyridin 18,5% (A); 2,5-Lutidin 5,1% (A); 3,5-Lutidin 0,6% (F); 2-Methyl-5-ethylpyridin 2,7% (A).

Beispiel 5

1140 ml einer 3,40molaren wäßrigen Lösung von Diammoniumhydrogenphosphat (pH 8,35) wurden in einem 2-Liter-Autoklaven auf 220°C erhitzt und bei 1500 UpM gerührt. In diese Lösung wurde innert 55 Min. kontinuierlich ein Gemisch aus 200,0 g Acetaldehyddiethylacetal und 105,5 g Formaldehyddimethylacetal eingepumpt (kalkulatorisches Molverhältnis Acetaldehyd zu Formaldehyd = 1 : 0,80). Dabei variierte der Reaktionsdruck zwischen 37 und 38 bar. Nach beendeter Zugabe des Aldehydgemisches wurde die Reaktionsmasse während 10 Min. bei 220°C weitergerührt und dann auf Raumtemperatur abgekühlt. Schließlich erfolgte eine Extraktion mit 3 · 100 ml Methylenchlorid sowie eine gaschromatographische Analyse der vereinigten Methylenchloridextrakte, wobei sich folgende Produkte mit den je nach Aldehyd-Bedarf auf eingesetztes Acetaldehyddiethylacetal (A) oder auf eingesetztes Formaldehyddimethylacetal (F) ergaben: Pyridin 1,0% (A); 3-Picolin 35,7% (F); 3-Ethylpyridin 34,4% (A); 2,5-Lutidin 7,4% (A); 3,5-Lutidin 0,3% (F); 2-Methyl-5-ethylpyridin 10,6% (A).

Beispiel 6

1140 ml einer 3,40molaren wäßrigen Lösung von Diammoniumhydrogenphosphat (pH 8,4) wurden in einem 2-Liter-Autoklaven auf 230°C erhitzt und bei 1500 UpM gerührt. In diese Lösung wurde mit einer ersten Pumpe 237,0 g Acetaldehyddiethylacetal und mit einer zweiten Pumpe ein Gemisch aus 38,8 g Hexamethylentetramin und 103 g Wasser kontinuierlich innerhalb 60 Min. eingespiesen (kalkulatorisches Molverhältnis Acetaldehyd zu Formaldehyd = 1 : 0,85). Dabei variierte der Reaktionsdruck zwischen 32 und 42 bar. Nach beendeter Zugabe des Aldehydgemisches wurde die Reaktionsmasse während 10 Min. bei 230°C weitergerührt und dann auf Raumtemperatur abgekühlt. Schließlich erfolgte eine Extraktion mit 3 · 100 ml Methylenchlorid sowie eine gaschromatographische Analyse der vereinigten Methylenchloridextrakte, wobei sich folgende Produkte mit den je nach Aldehyd-Bedarf auf eingesetztes Acetaldehyddiethylacetal (A) oder auf eingesetztes Hexamethylentetramin (F) bezogenen Ausbeuten ergaben: Pyridin 0,9% (A); 3-Picolin 57,7% (F); 3-Ethylpyridin 15,6% (A); 2,5-Lutidin 2,9% (A); 3,5-Lutidin 1,3% (F); 2-Methyl-5-ethylpyridin 1,2 (A).

**Patentansprüche**

1. Verfahren zur Herstellung von 3-Picolin, dadurch gekennzeichnet, daß man Acetaldehydacetale, gegebenenfalls im Gemisch mit Acetaldehyd bzw. dessen Polymeren und/oder Cortonaldehyd mit Formaldehydacetalen und/oder Hexamethylentetramin, gegebenenfalls im Gemisch mit Formaldehyd bzw. dessen Polymeren in wässeriger Phase bei einem Molverhältnis von Acetaldehydacetalen und gegebenenfalls Acetaldehyd zu Formaldehydacetalen und gegebenenfalls Formaldehyd von 1 : 0,5 bis 1 : 1,2 bzw. Crotonaldehyd zu Formaldehydacetalen, gegebenenfalls unter Zusatz von Formaldehyd von 1 : 1 bis 1 : 2,4 sowie Acetaldehydacetale, gegebenenfalls mit Acetaldehyd zu Hexamethylentetramin von 1 : 0,083 bis 1 : 0,2 bei Temperaturen von 180 bis 280°C im geschlossenen Gefäß in Gegenwart von Ammoniak und/oder Ammoniumionen und in Gegenwart von anorganischen oder organischen Säuren, die bei 20°C eine Dissoziationskonstante von $10^6$ bis $10^{-12}$ aufweisen, umsetzt.

2. Verfahren gemäß Patentanspruch 1, dadurch gekennzeichnet, daß man die Anionen der anorganischen und/oder organischen Säuren durch Zusatz der entsprechenden wasserlöslichen Alkali- und/oder Ammoniumsalze in die Reaktionslösung einbringt.

3. Verfahren gemäß Patentansprüchen 1 und 2, dadurch gekennzeichnet, daß man bei Temperaturen von 205 bis 240°C arbeitet.

4. Verfahren gemäß Patentansprüchen 1 bis 3, dadurch gekennzeichnet, daß man die Salze in wässeriger Lösung in einer Konzentration von 0,3 bis 10 Mol/l anwendet.

**Claims**

1. Process for the preparation of 3-picoline, characterized in that one one reacts acetaldehyde acetals, possibly in admixture with acetaldehyde or its polymers and/or crotonaldehyde, with formaldehyde acetals and/or hexamethylenetetramine, possibly in admixture with formaldehyde or its polymers, in aqueous phase at a mole ratio of acetaldehyde acetals and possibly acetaldehyde to formaldehyde acetals and possibly formaldehyde of 1 : 0,5 to 1 : 1,2 or of crotonaldehyde to formaldehyde acetals, possibly with the addition formaldehyde, of 1 : 1 to 1 : 2,4, as well as of acetaldehyde acetals, possibly with acetaldehyde, to hexamethylenetetramine of 1 : 0,083 to 1 : 0,2 at temperatures of 180 to 280°C in a closed vessel in the presence of ammonia and/or ammonium ions and in the presence of inorganic or organic acids which display a dissociation constant of $10^6$ to $10^{-12}$ at 20°C.

2. Process according to patent claim 1, characterized in that one introduces the anions of the inorganic and/or organic acids by the addition of the corresponding water-soluble alkali metal and/or ammonium salts to the reaction solution.

3. Process according to patent claims 1 and 2, characterized in that one operates at temperatures of 205 to 240°C.

4. Process according to patent claims 1 to 3, characterized in that one uses the salts in aqueous solution in a concentration of 0,3 to 10 mole/1.

**Revendications**

1. Procédé pour la préparation de la 3-picoline, caractérisé en ce que l'on fait réagir des acétals d'acétaldéhyde, éventuellement en mélange avec de l'acétaldéhyde ou ses polymères et/ou de l'aldéhyde crotonique avec des acétals de formaldéhyde et/ou de l'hexaméthylènetétramine, éventuellement en mélange avec du formaldéhyde ou ses polymères en phase aqueuse, à un rapport molaire des acétals d'acétaldéhyde et éventuellement de l'acétaldéhyde aux acétals de formaldéhyde et éventuellement au formaldéhyde, de 1 : 0,5 à 1 : 1,2, ou de l'aldéhyde crotonique aux acétals de formaldéhyde, éventuellement en ajoutant du formaldéhyde de 1 : 1 à 1 : 2,4, ainsi que des acétals d'acétaldéhyde, éventuellement avec de l'acétaldéhyde à l'hexaméthylènetétramine de 1 : 0,083 à 1 : 0,2, à des températures de 180 à 280°C, dans un récipient fermé en présence d'ammoniac et/ou d'ions d'ammonium, et en présence d'acides minéraux ou organiques qui présentent à 20°C une constante de dissociation de $10^6$ à $10^{-12}$.

2. Procédé selon la revendication 1, caractérisé en ce que l'on introduit les anions des acides minéraux et/ou organiques dans la solution de réaction par addition des sels alcalins et/ou d'ammonium solubles dans l'eau correspondants.

3. Procédé selon les revendications 1 et 2, caractérisé en ce que l'on travaille à des températures de 205 à 240°C.

4. Procédé selon les revendications 1 à 3, caractérisé en ce que l'on utilise les sels en solution aqueuse à une concentration de 0,3 à 10 moles/litre.